# EUROPEAN PATENT APPLICATION

(11) **EP 1 306 067 A1**
(43) Date of publication of application: **02.05.2003**
(21) Application number: 00970350.5
(22) Date of filing: 21.09.2000
(51) Int. Cl.: A61F 7/00

(54) **METHOD FOR DAMAGING THERMO NON-TOLERANCE PATHOLOGIC STRUCTURES OF A WARM-BLOODED ORGANISM**

(30) Priority: 21.07.2000 RU 2000119434
(71) Applicant: Suvernev, Alexey Vitalievich, Novosibirsk, 630008 (RU); Pisarev, Aleksandr Alekseevich, Novosibirsk, 630099 (RU); Vereschagin, Ivan Pavlovich, Novosibirsk, 630112 (RU); Efremov, Anatoly Vasilievich, Novosibirsk, 630049 (RU); Subbotin, Oleg Valentinovich, Novosibirsk, 630005 (RU)
(72) Inventor: Suvernev, Alexey Vitalievich, Novosibirsk, 630008 (RU); Pisarev, Aleksandr Alekseevich, Novosibirsk, 630099 (RU); Vereschagin, Ivan Pavlovich, Novosibirsk, 630112 (RU); Efremov, Anatoly Vasilievich, Novosibirsk, 630049 (RU); Subbotin, Oleg Valentinovich, Novosibirsk, 630005 (RU)
(74) Representative: Einsel, Martin
(86) International application number: RU0000376
(87) International publication number: WO02007655

(57) **Abstract**

The invention relates to medicine and can be used for oncology, reanimathology, neurology, narcology, virusology and dermatology and makes it possible to reduce the probability of complications. The inventive method consists in beating an organism by immerging said organism, apart from the head, in a thermal liquid, using a total anaesthesia, artificial long ventilation (ALV) and suppression of a total proteolytic activity in the blood. In order to heat the body a thermal liquid having a temperature ranging from 45 °C to 47 °C is used. The hearing process is carried out with forced convection of the liquid in such a way that the temperature in the gullet reaches 42.5 °C - 44 °C. Afterwards, the heating is discontinued and the patient is taken out of the thermal liquid. Suppression of total proteolytic activity in blood is carried out by controlling the velocity of the heating of the organism and by performing a constant infusion of a solution of at least one agent belonging to the antiproteinase class in an efficient quantity during the heating. The preferred velocity of the heating is equal to or not less than 1 °C per 5.5 minutes. A high-frequency ALV, providing a saturation ranging from 95 % to 100 %, is used.

## Description

### Field of the invention

The invention relates to medicine and can find application in oncology, reanimatology, neurology, narcology, virusology and dermatology.

### Prior art

There are known in the art methods for reproducing induced hyperthermia by total physical heating of the patient' s body on the background of narcosis and artificialung ventilation (ALV) which are used while treating oncologic diseases (Aleksandrov N.N., Savchenko N.E., et al. Use of hyperthermia and hyperglycemia while treating malignant tumors. ― M.: Meditsina, 1980, p.53-72). It is known a method for general heating of an organism (RU 2112471, publ. 10.06.98. Author ― Gleim G.K.). According to this method, the patient' s organism is heated with hot water on the background of a barbital anesthesia and ALV after a previous infusion of pyridoxine, hexamethylenetetramine, alpha-tocopherol, sodium hydroxybutyrate, and hexamethylenetetramine being additionally administered at the heating height.

The above mentioned method presents the following disadvantages:
1. Sodium hydroxybutyrate (GOMK) is used as one of the components, which is at present prohibited abroad due to its dangerous effects (transmineralization).
2. At the stage of the patient' s organism heating, artificial lung ventilation is performed in the mode of a traditional (16-20 per minute) volumetric pulmonary ventilation, which cannot satisfy the increased oxygen demand of the organism under the conditions of hyperthermia.
3. The process of the organism heating is "passive", its rate is not controlled, which can result in dangerous complications in patients having different body mass.
4. The measures of pharmacological protection applied (antioxidants, sodium hydroxybutyrate and reiterated infusion of hexamethylenetetramine) do not entirely prevent the negative effects of the heat aggression, which is attested by the need to submit the patients to ALV for 6 hours after a hyperthermia process.

The closest prior art to the inventive method is a method for damaging thermo non tolerance pathologic structures of a human organism by general controlled hyperthermia, comprising general heating of the patient' s organism by immersing said organism, apart from the head, in a thermal liquid such as hot water, using a total phentanylic anesthesia, antishock therapy, artificial lung ventilation and suppression of a total proteolytic activity in the blood by three-stage infusion of urotropine, the patient' s body heating being performed with hot water having constant temperature from 45 to 47'C to obtain in the gullet a temperature of 42.5 to 44'C and the rate of the body heating being equal to or not less than 1'C per 5 minutes by means of continuous forced convection of the heat carrier, and a high-frequency artificial lung ventilation is used (RU 2126667. A 61F 7/00, 27.02.99). Said method enables controland safety of the artificial general hyperthermia.
But said method shows a high probability of various complications occurring in the patient' s organism after the hyperthermia procedure, such as encephalopathy, DIC, cardiac rhythm disorders, hepatic insufficiency, etc.

### Essence of the invention

An object of the present invention is to make a method for general controlled hyperthermia with low probability of occurrence of postoperative complications.
A technical result of the present invention consists as well in reducing the probability of complications.
The technical result is obtained by the fact that in the method for damaging thermo non-tolerant\bio pathologic structures of a warm-blooded organism by a total controlled hyperthermia, consisting in heating an organism by immersing said organism, apart from the head, in a thermal liquid under the conditions of a forced convection of the same with the use of anesthetic means, antishock therapy and artificial lung ventilation and with the suppression of a total proteolytic activity in the blood, the heating is carried out at a thermal liquid temperature of 44 to 48'C until obtaining the organism temperature of 42.0 to 44.5'C, and the suppression of the total proteolytic activity in the blood is performed by controlling the organism heating rate and by carrying out a permanent infusion of at least one solution of an agent belonging to the antiproteinase class in an efficient amount during the heating.
Furthermore, the heating of the organism should be carried out at the rate of not less than 1'C per 5.5 minutes.
Moreover, as solutions for infusion, use is made of a hexamethylenetetramine solution at a rate of (2 to 6) mg/kg/min and of a pyridoxine solution at a rate of at least 0.2 mg\kg\min.
What is more, a high-frequency artificial lung ventilation providing for a saturation of 95 to 100% is used.

### Example of a preferred embodiment of the invention

The present method is performed as follows.
Before starting the hyperthermia procedure (for example, a day before performing the same), the universally adopted clinic and biochemical indices are examined in the patient, such as: blood hemoglobin and its formula, protein and its fraction percentage, sugar and the main blood electrolytes. The night before the procedure, premedication with the use of diazepam (10 mg) and, in drug addicts, of drugs in individual doses is performed. Immediately before the procedure (2-3 hours before), the patient is administered enterally, orally 20 ml of a 20% solution of hexamethylenetetramine (urotropine) and is injected intravenously 200 ml of 40% glucose solution at the rate of 0.5 to 1 g/kg/hr with insulin (0.1 unit/g of glucose). After that, infusion of rheopolyglukin (5 ml/kg) and a Ringer solution (10 ml/kg) with potassium chloride (7.5% - 20 ml), ascorbic acid (5% - 10.0 ml), pyridoxine (5%-10.0 ml) is carried out.

Induction of anesthesia is performed with hexenal (2.5% - 250 to 300 mg) or calypsol in a dose sufficient to induce the loss of consciousness (250 to 300 mg). After injecting a depolarizer-type myorelaxation drug, for example listenon in a dose of 3 to 5 mg/kg, the trachea is intubated and a high-frequency artificial lung ventilation (HF ALV) is started, for example, with an "Assistent-1""apparatus.
The HF ALV mode: breath pressure (P breath) is of 18 to 20 cm HrO, respiration rate (RR) is of 105 cycles per minute. The adequacy of HF ALV is monitored by pulsoxymetry, and the saturation level should be between 95 and 100%. Basic myorelaxation is provided by arduan (0.03 to 0.05 mg/kg). An electric resistance thermometer sensor is placed into the middle third of the gullet. After that, phentanyl (0.05% - 2 ml) is injected intravenously and an active physical heating of the patient' s body is started. For this purpose, the patient is immersed in a bath containing thermal liquid such as hot water at a temperature of (45 to 47'C), except the head. During the active heating, continuous infusion of hexamethylenetetramine (5 mg/kg/min) and pyridoxine (0.5 mg/kg/min) solutions is performed and intensive forced convection of the heat carrier is provided to maintain the rate of increase of the patient' s gullet temperature, i.e. the rate of heating the patient' s body being such which prevents proteolytic activity in the blood. A heating rate of at least 1'C per 5.5 minutes is optimal. The procedure is favored not only by water mixing in the bath but by the continuous infusion of said preparations. After the increase of temperature up to 42.0 ― 44.5'C, the patient is withdrawn out of the bath. HF ALV is continued and infusion of 200 ml of freshly defrosted plasma of the same group is made.
The temperature increase process is stopped, and as a result of the effects of air temperature in the operating rooT, of ice-bags on the head and of the HF ALV mode, a slow, passive recovery of the normal temperature is provided. After the body temperature decreases to 37'C, the patient is to be muffled up with blankets.
Diuresis occurrence (150-250 ml/hr), consciousness recovery at the temperature of 37'C and a possibility of stopping ALV even 60 to 90 minutes after the heating procedure demonstrate controlability and security of the method of total hyperthermia.

Thus, the clinically significant characteristics of the present invention are as follows:
1. The possibility to provide a controlled, fast rate of performing total hyperthermia up to 42.0-44.5'C without indices of a heat shock.
2. The additional efficient suppression of thermal proteolysis of the blood as a result of continuous infusion of hexamethylenetetramine and pyridoxine.
3. The reliable provision (owing to the HF ALV mode) of the organism oxygen demand which increases drastically under the hyperthermia conditions.
4. The occurrence in the patients, after the hyperthermia procedure, of rather fast consciousness recovery and of adequate quiet breathing without recourse to intensive treatment.
5. The absence of complications.

The high heating rate which prevents any increase of proteolytic activity in the blood while carrying out simultaneous and continuous infusion of antiproteinases inhibiting said process makes it possible to significantly lower a probability of postoperative complications such as encephalopathy, DIC, cardiac rhythm disorders, hepatic insufficiency, etc.
The present invention will be further illustrated by an example of medical treatment as follows.
A female patient aged 48 was received at the intensive treatment department of the Novosibirsk Hospital No 1 up to schedule. Diagnosis: cancer of the left mammary gland with a metastasis in the left lung.
Total controlled hyperemia (TCH) was carried out up to 43.6'C. A premedication with diazepam (10 mg) was performed immediately before starting the total heating, urotropine (20% - 20 ml) was administered enterally, infusion was made with 200 ml of a 40% solution of glucose with insulin, 400 ml of rheopolyglukin, 500 ml of Ringer solution with potassium chloride (7.5% - 20 ml), ascorbic acid and pyridoxine (10 ml of a 5% solution for both).
Induction of anesthesia was performed with thiopental and calypsol. Relaxants were administered, the trachea was intubated, the HF ALV was carried out at a mode providing saturation at the level of 99-100%. Total anesthesia was maintained at the level 111 by fractional intravenous transfusion of phentanyl.

The patient' s body heating was carried out in a water bath (46'C) at a rate of 1'C per 2 minutes. Continuous infusion of hexamethylenetetramine (5 mg/kg/min) and pyridoxine (0.5 mg/kg/min) solutions was performed in parallel. During the heating process, after occurrence of the gullet temperature of 43.6'C, the heating was stopped.
The patient was extracted from the bath. Ice-bags were placed onto the head. 450 ml of defrosted plasma and 150 ml of a 10% glucose solution with insulin (10 units) and potassium chloride (10 ml) were transfused intravenously. 60 minutes after the temperature normalization, the patient was extubated.
On the next day, the patient was active, she had no complaints and she took care 10 of herself. There was no need to carry out intensive treatment. 9 days later, the patient was operated: a metastasis ablation in the bottom part of the left lung and a sector resection of the left mammary gland were performed. Histology study of the ablated tissue samples showed a manifested, up to necrosis, pathomorphism of cancer cells.
A month later, this patient was submitted, without any complications, to a eiterated total controlled hyperthermia (43.5'C) as a method of an antirecidivating treatment.

### Industrial use

The method of the present invention is applicable under the conditions of a common hospital with the use of wide-spread preparations and equipment. It is efficient while treating various diseases, even such serious ones like narcomania, neglected forms of cancer and HIV-infection. It is controlled, sure and has a low probability of complications.

## Claims

1. A method for damaging thermo non-tolerant pathol+ogic biostructures of an organism by total controlled hyperthermia, consisting in heating the organism by immersing it, apart from the head, in a thermal liquid under the conditions its forced convection with the use of anesthetic means, means for antishock therapy and artificial lung ventilation, and with suppression of the total proteolytic activity in the blood, wherein said heating is performed at the thermal liquid temperature of 44 to 48'C until obtaining the organism temperature of 42.0 to 44.5'C, said suppression of the total proteolytic activity in the blood being performed by controlling the organism heating rate and by carrying out a continuous infusion of at least one solution of an agent blonging to the antiproteinase class in an efficient amount during the heating.

2. A method as claimed in claim 1, wherein said heating of the organism is carried out at a rate of not less'than 1'C per 5.5 minutes.

3. A method as claimed in claim 1 or 2, wherein said solutions for infusion represent a hexamethylenetetramine solution at a rate of (2 to 6) mg/kg/min ad a pyridoxine solution at a rate at least 0.2 mg/kg/min.

4. A method as claimed in any claim 1, 2 or 3, wherein a high-frequency artificial lung ventilation providing for a saturation of 95 to 100% is used.
